# EUROPÄISCHE PATENTSCHRIFT

(11) **EP 4 220 100 B1**
(45) Veröffentlichungstag und Bekanntmachung des Hinweises auf die Patenterteilung: **28.10.2026**
(21) Anmeldenummer: 22153537.0
(22) Anmeldetag: 26.01.2022
(51) Int. Cl.: A61B 5/097, A61B 5/00, A61B 5/087, A61M 5/00, A61M 15/08, A61M 11/00, A61M 15/00, G01F 1/34, G01F 1/684, G01F 1/69, G01F 1/74, G01N 33/497

(54) **LUNGENDEPOSITIONSANALYSE**
PULMONARY DEPOSITION ANALYSIS
ANALYSE DES DÉPÔTS PULMONAIRES

(43) Veröffentlichungstag der Anmeldung: 02.08.2023
(73) Patentinhaber: Pulmotree Medical GmbH, 80333 München (DE)
(72) Erfinder: Krüger, Ulf, 80799 München (DE); Förner, Pascal, 96346 Wallenfels (DE)
(74) Vertreter: Stolmár & Partner Patentanwälte PartG mbB

(56) Entgegenhaltungen:
- EP-B1- 0 667 168
- US-A1- 2007 157 931
- US-A1- 2010 282 247

## Beschreibung

### Gebiet der Erfindung

Die Erfindung betrifft ein Analysesystem zur Depositionsbestimmung eines Aerosols für einen Atmungsapparat, ein Verfahren zur Depositionsbestimmung eines Aerosols für einen Atmungsapparat und eine Verwendung eines solchen Analysesystems zur Durchführung eines solchen Verfahrens.

### Stand der Technik

Es existieren Medizingeräte, mit deren Hilfe Medikamente in den Atmungsapparat eines Patienten eingebracht werden können. Derartige Medizingeräte sind beispielsweise Inhalatoren, die wiederum in Dosierinhalatoren (Druckgas oder Normaldruck), Pulverinhalatoren, Soft Mist-Inhalatoren beziehungsweise Soft-Inhalatoren und Vernebler unterteilt werden.

Mit Verneblern werden feine Flüssigkeitströpfchen von einem flüssigen Medikamenteneservoir abgetrennt. Dadurch entsteht ein Aerosol, das durch ein Mundstück vom Patienten eingeatmet werden kann. Je nach verwendetem Funktionsprinzip werden Vernebler in Düsenvernebler, Ultraschallvernebler oder Schwingmembranvernebler (Meshvernebler) unterteilt.

Generell werden mit solchen Medizingeräten Medikamentenaerosole einem vom Patienten aufgenommenen Luftstrom zugegeben. Über Körperöffnungen des Patienten, beispielsweise dessen Mund oder dessen Nase gelangen die Medikamentenaerosole dann in den Atmungsapparat des Patienten, in dem sie anschließend zumindest teilweise abgelagert werden.

Der Ort der Deposition beziehungsweise Ablagerung von Medikamenten beziehungsweise Medikamentenaerosolen im Atmungsapparat, beispielsweise in der Lunge ist von unterschiedlichen Variablen abhängig, zu denen beispielsweise neben der Aerosolcharakteristik (Durchmesser beziehungsweise Partikelgröße und Durchmesserverteilung beziehungsweise Partikelgrößenverteilung) die Inhalationsgeschwindigkeit beziehungsweise der Inhalationsfluss (Volumenstrom) und das eingeatmete Volumen gehören. Eine Änderung dieser Variablen beeinflusst den Depositionsort des Aerosols. Das Aerosol kann beispielsweise in der Lungenperipherie, bronchial, oder im Pharynxbereich abgeschieden werden. Da jedes Medikament jedoch für einen bestimmten Wirk- beziehungsweise Depositionsort vorgesehen ist beziehungsweise an diesem bestmöglich seine Wirkung entfalten kann, gilt es, diesen Wirkort bestmöglich beziehungsweise mit größtmöglicher Effizienz zu erreichen. Der Depositionsort von Medikamentenaerosolen im Atmungsapparat ist aktuell nur durch klinische Depositionsstudien bestimmbar. Diese basieren auf bildgebenden Verfahren, beispielsweise auf der Röntgendiagnostik oder der Szintigrafie und sind daher sehr aufwendig. Es existieren demgegenüber Modelle zur Berechnung des Depositionsortes von Medikamentenaerosolen im Atmungsapparat, wobei diese auf standardisierten Flusskurven, Annahmen zur Aerosolcharakteristik und Ergebnissen aus klinischen Depositionsstudien basieren, wodurch lediglich eine theoretische Annäherung erfolgen kann. So wird beispielsweise nicht berücksichtigt, wie der Patient bei der jeweiligen Inhalation tatsächlich inhaliert hat. EP 0 667 166 B1 beschreibt ein solches Inhalationstrainingsgerät zur Maximierung der Zufuhr eines inhalierbaren Medikaments zu den Rezeptoren in der Lunge eines Patienten.

Dementsprechend ist die Bestimmung der Deposition von Medikamentenaerosolen im Atmungsapparat entweder sehr aufwendig oder sehr ungenau.

### Darstellung der Erfindung

Aufgabe der vorliegenden Erfindung ist daher die Bereitstellung eines Systems und eines Verfahrens, mit dem eine einfache und genaue Dosierung von Medikamentenaerosolen ermöglicht wird.

Diese Aufgabe wird durch ein Analysesystem nach Anspruch 1, ein Verfahren nach Anspruch 7 und eine Verwendung eines Analysesystems zur Durchführung eines Verfahrens nach Anspruch 14 gelöst. Weitere, die Erfindung ausgestaltende Merkmale sind in den Unteransprüchen enthalten.

Ein erfindungsgemäßes Analysesystem zur Depositionsbestimmung eines Aerosols für einen Atmungsapparat hat eine Analyseeinrichtung aufweisend eine Luftführung, einen ersten Luftsensor, eine Steuereinrichtung und eine Applikationskomponente, wobei die Luftführung dazu eingerichtet ist, Luft entlang des ersten Luftsensors zu der Applikationskomponente und umgekehrt zu führen, der erste Luftsensor dazu eingerichtet ist, zumindest einen ersten Luftströmungsparameter der an dem ersten Luftsensor entlang geführten Luft zu erfassen, und die Steuereinrichtung dazu eingerichtet ist, den ersten Luftsensor derart zu steuern, dass ein zeitlicher Verlauf des ersten Luftströmungsparameters als ein erstes Messergebnis messbar ist, und das erste Messergebnis zu einer Auswerteeinrichtung übertragbar ist. Derart wird eine einfache und genaue Bestimmung der Deposition eines Aerosols für einen Atmungsapparat ermöglicht, also Rückschlüsse darauf, inwieweit, in welchem Umfang und an welchem Ort über die Applikationskomponente bei einer Anwendung des erfindungsgemäßen Analysesystems Aerosole in dem Atmungsapparat deponiert wurden. Dadurch wird wiederum eine genaue Dosierung von Medikamentenaerosolen ermöglicht. Der Atmungsapparat ist ein Bestandteil eines Organismus, mit dem Luft aus der Umgebung aufgenommen und Luft aus demselben an die Umgebung abgegeben werden kann. Die Auswerteeinrichtung ist dazu geeignet, das erste Messergebnis zu empfangen. Dafür kann eine zusätzliche Empfangskomponente vorgesehen sein.

Erfindungsgemäß weist das Analysesystem die Auswerteeinrichtung auf, und ist die Auswerteeinrichtung dazu eingerichtet, anhand des ersten Messergebnisses, zumindest eines Depositionsparameters, und eines Depositionsmodells eine Depositionsbestimmung durchzuführen und als Ergebnis der Depositionsbestimmung ein Depositionsergebnis auszugeben. Es wird derart ermöglicht, pharmakokinetische Prozesse zu modellieren, wodurch eine gezieltere und wirksamere Behandlung ermöglicht wird. Das Depositionsergebnis zeigt an, in welchem Umfang und an welcher Position in dem Atmungsapparat Medikamentenaerosole deponiert beziehungsweise abgelagert wurden. Anhand des Depositionsergebnisses können dann Rückschlüsse darauf gezogen werden, wie die Medikamentenaerosole resorbierbar sind.

Vorzugsweise ist die Auswerteeinrichtung dazu eingerichtet, die Depositionsbestimmung unabhängig von einer Netzwerkverbindung durchzuführen. Auf diese Weise wird ein besserer beziehungsweise vereinfachter Schutz von Patientendaten ermöglicht. Vorzugsweise ist die Steuereinrichtung ferner dazu eingerichtet, das erste Messergebnis zu speichern, und ist das erste Messergebnis von der Steuereinrichtung zu der Auswerteeinrichtung übertragbar. Derart wird es ermöglicht, die Depositionsbestimmung nach einer abgeschlossenen Messung durchzuführen, wodurch eine robuste und unabhängige Auswertung ermöglicht wird.

Vorzugsweise ist die Steuereinrichtung ferner dazu eingerichtet, den ersten Luftsensor derart zu steuern, dass eine Vielzahl aufeinanderfolgender Teilmessergebnisse messbar sind. Die Teilmessergebnisse können einzeln und gebündelt zu der Auswerteeinrichtung übertragbar sein. Derart wird es ermöglicht, die Depositionsbestimmung während einer Messung beziehungsweise Reihe von Messungen durchzuführen und das Depositionsergebnis ständig zu aktualisieren, wodurch dem Patienten unmittelbar Anwendungsinformationen zur Verfügung gestellt werden können. Es wird dadurch ferner ermöglicht, die Abgabe von Medikamentenaerosolen temporär zu pausieren, beispielsweise wenn anhand eines Teilmessergebnisses festgestellt wird, dass die Deposition des Medikamentenaerosols nicht wie gewünscht oder potentiell sogar in schädigender Weise erfolgen würde. Dadurch wird auch der Patientenschutz verbessert.

Vorzugsweise weist das Analysesystem ferner einen zweiten Luftsensor auf, der in der Luftführung angeordnet ist, und ist die Steuereinrichtung ferner dazu eingerichtet, den zweiten Luftsensor derart zu steuern, dass ein zeitlicher Verlauf des zweiten Luftströmungsparameters als ein zweites Messergebnis messbar ist. Das zweite Messergebnis ist zu der Auswerteeinrichtung übertragbar, und die Auswerteeinrichtung ist ferner dazu eingerichtet, die Depositionsbestimmung zusätzlich anhand des zweiten Messergebnisses durchzuführen. Es ist ferner möglich, dass das Analysesystem einen dritten beziehungsweise eine Vielzahl von Luftsensoren aufweist, sodass ein drittes beziehungsweise eine Vielzahl von Messergebnissen messbar ist beziehungsweise sind. Derart wird es ermöglicht, eine genauere Depositionsbestimmung durchzuführen.

Vorzugsweise ist der erste Luftsensor ferner dazu eingerichtet, einen zweiten Luftströmungsparameter der an dem ersten Luftsensor entlanggeführten Luft zu erfassen, und ist die Steuereinrichtung ferner dazu eingerichtet, den ersten Luftsensor derart zu steuern, dass ein zeitlicher Verlauf des zweiten Luftströmungsparameters als ein zweites Messergebnis messbar ist. Das zweite Messergebnis ist zu der Auswerteeinrichtung übertragbar, und die Auswerteeinrichtung ist ferner dazu eingerichtet, die Depositionsbestimmung zusätzlich anhand des zweiten Messergebnisses durchzuführen. Derart wird es ermöglicht, eine genauere Depositionsbestimmung durchzuführen.

Vorzugsweise weist das Analysesystem ferner einen Inhalator auf, wobei der Inhalator insbesondere ein Vernebler, besonders bevorzugt ein Meshvernebler ist. Derart wird es ermöglicht, im Zuge einer Inhalation von beispielsweise Medikamentenaerosolen durch einen Patienten, eine Depositionsbestimmung ohne zusätzliche Geräte durchzuführen, wodurch die Handhabung vereinfacht wird.

Ein erfindungsgemäßes Verfahren zur Depositionsbestimmung eines Aerosols für einen Atmungsapparat weist die Schritte des (i.) Führens von Luft in einer Luftführung entlang eines ersten Luftsensors zu einer Applikationskomponente, (ii.) Erfassens zumindest eines ersten Luftströmungsparameters der an dem ersten Luftsensor entlang geführten Luft mittels des ersten Luftsensors, (iii.) Steuerns des ersten Luftsensors mittels einer Steuereinrichtung derart, dass ein zeitlicher Verlauf des ersten Luftströmungsparameters als ein erstes Messergebnis gemessen wird, (iv.) Durchführens einer Depositionsbestimmung anhand des ersten Messergebnisses, zumindest eines Depositionsparameters und eines Depositionsmodells, und (v.) Ausgebens eines Depositionsergebnisses als Ergebnis der während Schritt iv. durchgeführten Depositionsbestimmung auf, wobei das Depositionsergebnis anzeigt, in welchem Umfang und an welcher Position in dem Atmungsapparat Medikamentenaerosole deponiert wurden. Die Depositionsbestimmung bezieht sich auf einen Bereich beziehungsweise Bereiche und den Umfang der an dem Bereich beziehungsweise an den Bereichen abgelagerten Aerosole. Derart wird eine einfache und genaue Bestimmung der Deposition eines Aerosols für einen Atmungsapparat ermöglicht, wodurch wiederum eine genaue Dosierung von Medikamentenaerosolen ermöglicht wird.

Ferner können mit Hilfe des Depositionsergebnisses Rückschlüsse darauf gezogen werden, ob der anvisierte beziehungsweise gewünschte Depositionsort mit dem realen Depositionsort, also mit dem, der über das Depositionsergebnis ausgegeben wird, übereinstimmt. Derart können zusätzlich Rückschlüsse bezüglich Abweichungen beziehungsweise Ausprägungen von Vitalparametern beziehungsweise patientenspezifischen Parametern, beispielsweise der Blutplasmawirkstoffkonzentration, gezogen werden.

Vorzugsweise sind die Aerosole Medikamentenaerosole, die einem Patienten über dessen Atmungsapparat verabreichbar sind. Derart können auf einfache Art und Weise Rückschlüsse auf gegebenenfalls therapiebegünstigende Gegebenheiten gezogen werden.

Vorzugsweise wird zwischen den Schritten iii. und iv. das erste Messergebnis mittels einer Auswerteeinrichtung empfangen und Schritt iv. mittels der Auswerteeinrichtung durchgeführt. Die oben beschriebenen Aspekte in Bezug auf die Auswerteeinrichtung treffen hier analog zu.

Vorzugsweise wird während Schritt iii. das erste Messergebnis nach der Messung in der Steuereinrichtung gespeichert und anschließend von der Steuereinrichtung zu der Auswerteeinrichtung übertragen. Derart wird es ermöglicht, die Depositionsbestimmung nach einer abgeschlossenen Messung durchzuführen, wodurch eine robuste und unabhängige Auswertung ermöglicht wird.

Vorzugsweise wird während Schritt iii. der erste Luftsensor mittels der Steuereinrichtung derart gesteuert, dass eine Vielzahl aufeinanderfolgender Teilmessergebnisse gemessen werden. Die oben beschriebenen Aspekte in Bezug auf die Teilmessergebnisse treffen hier analog zu.

Vorzugsweise wird während Schritt iv. zu einer Vielzahl aufeinander folgender Zeitpunkte jeweils mit den bis zu dem aktuellen Zeitpunkt gemessenen Teilmessergebnissen eine Depositionsbestimmung durchgeführt und während Schritt v. zu der Vielzahl aufeinander folgender Zeitpunkte jeweils mit den bis zu dem aktuellen Zeitpunkt während Schritt iii. gemessenen Teilmessergebnissen ein aktuelles Depositionsergebnis ausgegeben. Die oben beschriebenen Aspekte in Bezug auf die Möglichkeit der Durchführung der Depositionsbestimmung während einer Messung beziehungsweise Reihe von Messungen sowie Rückmeldung an den Patienten während einer Inhalation treffen hier analog zu. Die bis zu dem aktuellen Zeitpunkt gemessenen Teilmessergebnisse werden beziehungsweise wurden dabei gegebenenfalls an die Auswerteeinrichtung übermittelt.

Vorzugsweise wird während Schritt i. ferner Luft entlang eines zweiten Luftsensors zu der Applikationskomponente geführt, während Schritt ii. ferner ein zweiter Luftströmungsparameter der an dem zweiten Luftsensor entlang geführten Luft mittels des zweiten Luftsensors erfasst, während Schritt iii. ferner der zweite Luftsensor mittels der Steuereinrichtung derart gesteuert, dass ein zeitlicher Verlauf des zweiten Luftströmungsparameters als ein zweites Messergebnis gemessen wird, und während Schritt iv. die Depositionsbestimmung zusätzlich anhand des zweiten Messergebnisses durchgeführt. Die oben beschriebenen Aspekte in Bezug auf den zweiten Luftsensor und das zweite Messergebnis treffen hier analog zu. Gegebenenfalls wird das zweite Messergebnis zu der Auswerteeinrichtung übertragen und zwischen den Schritten iii. und iv. ferner das zweite Messergebnis mittels der Auswerteeinrichtung empfangen.

Vorzugsweise wird während Schritt ii. ferner ein zweiter Luftströmungsparameter der an dem ersten Luftsensor entlanggeführten Luft mittels des ersten Luftsensors erfasst, während Schritt iii. ferner der erste Luftsensor mittels der Steuereinrichtung derart gesteuert, dass ein zeitlicher Verlauf des zweiten Luftströmungsparameters als ein zweites Messergebnis gemessen wird, und während Schritt iv. die Depositionsbestimmung zusätzlich anhand des zweiten Messergebnisses durchgeführt. Die oben beschriebenen Aspekte in Bezug auf die Eignung des ersten Luftsensors, einen zweiten beziehungsweise dritten Luftströmungsparameter beziehungsweise eine Vielzahl von Luftströmungsparametern zu erfassen, treffen hier analog zu. Gegebenenfalls wird am Ende des Schrittes ii. das zweite Messergebnis zu der Auswerteeinrichtung übertragen, und zwischen den Schritten iii. und iv. ferner das zweite Messergebnis mittels der Auswerteeinrichtung empfangen.

Eine erfindungsgemäße Verwendung eines erfindungsgemäßen Analysesystems zur Durchführung eines erfindungsgemäßen eines Verfahrens findet insbesondere für eine menschliche Lunge statt. Derart wird eine einfache und genaue Bestimmung der Deposition eines Aerosols für einen Atmungsapparat ermöglicht, wodurch wiederum eine genaue Dosierung von Medikamentenaerosolen ermöglicht wird.

### Kurze Beschreibung der Figuren

Fig. 1a zeigt eine abstrakte Darstellung eines bevorzugten Analysesystems mit einem ersten Luftsensor.
Fig. 1b zeigt eine abstrakte Darstellung eines bevorzugten Analysesystems mit einem ersten Luftsensor und einem zweiten Luftsensor.
Fig. 1c zeigt eine abstrakte Darstellung des in Fig. 1a dargestellten Analysesystems mit einem Inhalator.
Fig. 2 zeigt eine abstrakte Darstellung des in Fig. 1c dargestellten Analysesystems mit einem Atmungsapparat.
Fig. 3 zeigt ein bevorzugtes Verfahren.
Fig. 4a zeigt ein bevorzugtes Depositionsergebnis.
Fig. 4b zeigt ein weiteres bevorzugtes Depositionsergebnis.
Fig. 5 zeigt einen zeitlichen Verlauf eines ersten Luftströmungsparameters als ein bevorzugtes erstes Messergebnis.
Fig. 6a zeigt eine Kopfeinheit mit Applikationskomponente in einer seitlichen Schnittansicht mit eingezeichneter Luftströmung.
Fig. 6b zeigt die in Fig. 6a gezeigte Kopfeinheit in einer Schnittansicht von unten.

### Beschreibung der bevorzugten Ausführungsformen

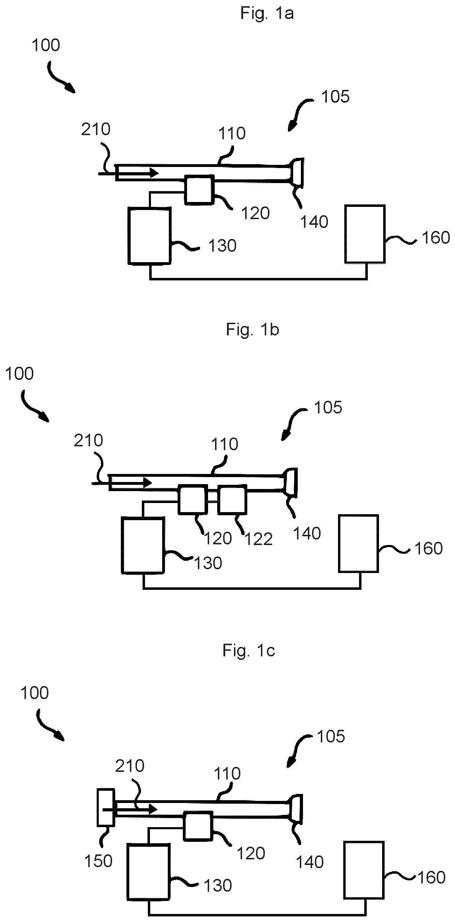

Fig. 1a zeigt eine abstrakte Darstellung eines bevorzugten Analysesystems 100 mit einem ersten Luftsensor 120. Das Analysesystem 100 besteht aus einer Analyseeinrichtung 105. Die Analyseeinrichtung 105 weist eine Luftführung 110 auf. Durch die Luftführung 110 ist Luft 210 führbar. Die Führung der Luft 210 erfolgt in der in Fig. 1a gezeigten Darstellung vom linken Ende zu einer am rechten Ende angeordneten Applikationskomponente 140. Die Führung der Luft 210 kann jedoch auch anders herum gerichtet erfolgen. Der erste Luftsensor 120 ist derart angeordnet, dass die in der Luftführung 110 geführte Luft 210 an dem ersten Luftsensor 120 entlang geführt wird. An den ersten Luftsensor 120 ist eine Steuereinrichtung 130 angeschlossen. An die Steuereinrichtung 130 ist wiederum eine Auswerteeinrichtung 160 angeschlossen. Die Verbindung zwischen Steuereinrichtung 130 und erstem Luftsensor 120 kann wie dargestellt kabelgebunden, aber auch kabellos vorgesehen sein, sodass eine kabelgebundene oder kabellose Signalübertragung ermöglicht wird. Ferner können der erste Luftsensor 120 und die Steuereinrichtung 130 in einem Bauteil zusammengefasst sein. Des Weiteren können die Applikationskomponente 140 und die Luftführung 110 als einstückiges Bauteil ausgeführt sein.

Der erste Luftsensor 120 ist beispielsweise ein Luftdrucksensor, sodass als erster Luftströmungsparameter der Luftdruck messbar ist, ein Luftmassensensor, sodass als erster Luftströmungsparameter die Luftmasse messbar ist, ein Luftmengensensor, sodass als erster Luftströmungsparameter die Luftmenge beziehungsweise der Inhalationsfluss messbar ist oder ein Luftgeschwindigkeitssensor, sodass als erster Luftströmungsparameter die Luftgeschwindigkeit beziehungsweise die Inhalationsgeschwindigkeit messbar ist. Es sind ferner weitere Luftsensortypen möglich, mit denen weitere Luftströmungsparameter messbar sind, beispielsweise die Lufttemperatur und die Luftfeuchte. Als solche weiteren Luftströmungsparameter sind nicht nur fluidmechanische Parameter denkbar, sondern auch allgemeine Parameter in Bezug auf die Luft der Luftströmung.

Mit Messung der Inhalationsgeschwindigkeit beziehungsweise des Inhalationsflusses als Luftströmungsparameter wird eine kontinuierliche Berechnung des Volumenstroms ermöglicht. Neben der Durchführung einer Depositionsanalyse können anhand beziehungsweise mit Hilfe der Luftströmungsparameter weitere Aktionen eingeleitet werden. Werden beispielsweise bestimmte Grenzwerte des Volumenstroms überbeziehungsweise unterschritten, können automatisch bestimmte Aktionen des Analysesystems 100 ausgelöst werden. So kann beispielsweise ein entsprechendes Steuersignal erzeugt werden, das dafür sorgt, dass eine an das Analysesystem 100 angegliederte Aerosolerzeugung ein- oder ausgeschaltet wird beziehungsweise pausiert wird. Des Weiteren kann ein entsprechendes Steuersignal erzeugt werden, das dafür sorgt, dass dem Anwender beispielsweise eines an das Analysesystems angegliederten Inhalators 150 (siehe Fig. 1c) ein Feedback zum Gebrauchsverhalten gibt. Das Feedback kann haptisch, visuell oder akustisch sein und auch appbasiert in Verbindung mit einem mobilen Endgerät vermittelt werden.

Weitere mögliche Luftströmungsparameter sind das inspiratorische Volumen (Inspired Volume, IV), der inspiratorische Maximalfluss (Peak Inspiratory Flow, PIF) und der Atemwegswiderstand (Airway Resistance, RAW).

Die Luftführung 110 kann im einfachsten Fall ein gerades Rohr sein, aber auch ein gebogenes Rohr, ein Rohr mit variablem Durchmesser, ein komplexes verzweigtes System oder jedes andere System, mit dem Luft auf eine bevorzugte Weise geführt werden kann. Die Luftführung 110 kann insbesondere derart ausgebildet sein, dass die Luft als Nebenstrom beziehungsweise in einem Nebenarm entlang des ersten Luftsensors 120 geführt wird. Das birgt den Vorteil, dass das Risiko einer Kontamination reduziert werden kann. Im Falle des Führens der Luft als Nebenstrom kann ein Nebenstrommessprinzip angewandt werden, sodass bei Durchführung einer Depositionsbestimmung anhand des ersten Messergebnisses die Charakteristika der für die Berechnung des Depositionsortes relevanten Hauptströmung anstatt der Nebenströmung berücksichtigt werden.

In dem Analysesystem 100, beispielsweise in der Auswerteeinrichtung 160 kann ein Depositionsmodell hinterlegt sein. Das Depositionsmodell kann algorithmisch hinterlegt sein. Das Depositionsmodell kann empirische Elemente und solche, die auf mathematischen beziehungsweise physikalischen Modellen beruhen, aufweisen, vollständig empirisch basiert sein oder vollständig auf mathematischen beziehungsweise auf physikalischen Modellen basieren.

Ein mögliches Depositionsmodell ist beispielsweise das "Human Respiratory Tract Model for Radiological Protection" der Internationalen Strahlenschutzkommission ("ICRP 66"). Es existieren weitere Depositionsmodelle kommerzieller Natur, die teilweise deutlich genauer als das Depositionsmodell ICRP 66 sind. Beispielsweise existieren kommerzielle Depositionsmodelle, die speziell auf pharmazeutische Aerosole ausgerichtet sind.

Fig. 1b zeigt eine abstrakte Darstellung eines bevorzugten Analysesystems 100 mit einem ersten Luftsensor 120 und einem zweiten Luftsensor 122. Das in Fig. 1b dargestellte Analysesystem 100 unterscheidet sich von dem in Fig. 1a dargestellten Analysesystem 100 dadurch, dass es einen zweiten Luftsensor 122 aufweist. Der zweite Luftsensor 122 ist über den ersten Luftsensor 120 mit der Steuereinrichtung 130 und über die Steuereinrichtung 130 mit der Auswerteeinrichtung 160 verbunden. Der zweite Luftsensor 122 kann auch direkt mit der Steuereinrichtung 130 verbunden sein.

Die Analyseeinrichtung 105 kann ferner weitere Sensoren aufweisen, insbesondere Lufttemperatur- und Luftfeuchtesensoren.

Die Auswerteeinrichtung 160 kann wie dargestellt als physischer Bestandteil des Analysesystems 100, aber auch von diesem getrennt vorgesehen sein, beispielsweise als in eine Cloud ausgelagert.

Die Auswerteeinrichtung 160 kann ferner auch mehrgeteilt vorgesehen sein, beispielsweise zweigeteilt, wobei mit jedem Teil der Auswerteeinrichtung 160 ein Teil der Depositionsbestimmung durchgeführt wird. Auch die Ausgabe des Depositionsergebnisses kann an verschiedenen Endgeräten erfolgen.

Mittels des ersten Luftsensors 120 und des zweiten Luftsensors 122 kann es beispielsweise ermöglicht werden, den Luftdruck an verschiedenen Positionen beziehungsweise zu unterschiedlichen Zeitpunkten zu ermitteln, sodass ein zeitlich bedingter beziehungsweise örtlich bedingter Differenzdruck für die Depositionsbestimmung verwendbar ist. Es ist ferner möglich, beispielsweise sowohl Luftdruck als auch Luftgeschwindigkeit zu messen oder jede andere mögliche Kombination unterschiedlicher Luftströmungsparameter. Tendenziell kann durch eine höhere Anzahl an Luftsensoren 120, 122 eine höhere Messgenauigkeit erreicht werden.

Es ist ferner möglich, dass der erste Luftsensor 120 dazu eingerichtet ist, einen dritten beziehungsweise eine Vielzahl von Luftströmungsparametern zu erfassen, sodass ein drittes beziehungsweise eine Vielzahl von Messergebnissen messbar ist beziehungsweise sind. Es ist ebenfalls möglich, dass eine Kombination aus mehreren Luftsensoren 120, 122 verwendet wird, die jeweils dazu eingerichtet sind, einen oder mehrere Luftströmungsparameter zu erfassen.

Fig. 1c zeigt eine abstrakte Darstellung des in Fig. 1a dargestellten Analysesystems 100 mit einem Inhalator 150. Der Inhalator 150 ist auf der linken Seite der Luftführung 110 angeordnet. In der dargestellten Anordnung ist aus dem Inhalator 150 stammende Luft 210 durch die Luftführung 110 führbar, wobei die Luft 210 im Inhalator 150 mit Aerosolen, insbesondere mit Medikamentenaerosolen anreicherbar ist.

Der Inhalator 150 kann beispielsweise ein Dosierinhalator (Metered Dose Inhaler beziehungsweise MDI), ein Pulverinhalator, ein Soft Mist-Inhalator beziehungsweise Soft-Inhalator oder ein Vernebler sein. Der Vernebler kann ein Düsenvernebler, ein Ultraschallvernebler oder ein Schwingmembranvernebler (Meshvernebler) sein.

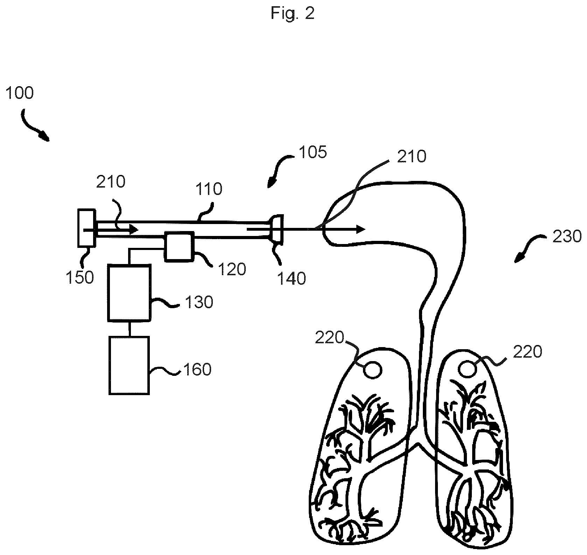

Fig. 2 zeigt eine abstrakte Darstellung des in Fig. 1c dargestellten Analysesystems 100 mit einem Atmungsapparat 230. Das Analysesystem 100 ist dabei in Bezug auf den Atmungsapparat 230 derart angeordnet, dass die Luft 210 über die Applikationskomponente 140 in den Atmungsapparat 230 führbar ist. Der dargestellte Atmungsapparat 230 ist ein menschlicher Atmungsapparat. In dem Atmungsapparat 230 sind mittig jeweils links und rechts Aerosole 220 angeordnet beziehungsweise deponiert. Das Aerosol 220 stammt aus dem Inhalator 150, kann aber auch aus einem Beatmungsgerät stammen, also oral appliziert werden. Im Falle der oralen Applikation mittels eines Beatmungsgerätes wird das Aerosol 220 direkt in dem Beatmungsgerät generiert. Das Analysesystem 100 kann dann direkt in das Beatmungsgerät integriert sein. Das Aerosol 220 kann ebenfalls nasal appliziert werden. Dementsprechend kann die Applikationskomponente 140 beispielsweise ein Mundstück oder eine Atemmaske für den Inhalator 150, aber auch ein Nasenaufsatz für die nasale Applikation oder eine Atemmaske für ein Beatmungsgerät sein.

Stammt das Aerosol 220 aus einem Beatmungsgerät, können auf in diesem gemessenen weiteren Luftströmungsparametern basierende Messergebnisse ebenfalls zu der Auswerteeinrichtung 160 übertragbar sein. Auf diese Weise kann beziehungsweise können der vom Beatmungsgerät vorgegebene Atemzyklus beziehungsweise die am Beatmungsgerät eingestellten Beatmungsparameter bei der Auswertung zusätzlich berücksichtigt werden. Es ist ebenfalls möglich, dass ein zusätzliches Gerät zur kontrollierten Inhalation verwendet wird, von dem dann analog zum Beatmungsgerät Parameter des Atemzyklus beziehungsweise eingestellte Inhalationsparameter bei der Auswertung zusätzlich berücksichtigt werden können. Dasselbe ist für zusätzliche Geräte zur kontrollierten nasalen Applikation denkbar.

Der Atmungsapparat 230 kann ein menschlicher Atmungsapparat sein und die Nase, die Nasennebenhöhlen, den Rachen, den Kehlkopf, die Luftröhre, den linken und rechten Luftröhrenhauptast, den Stammbronchus (Bronchus principalis), die Bronchien, die Bronchiolen, die Alveolargänge sowie die Lungenbläschen (Alveolen) umfassen.

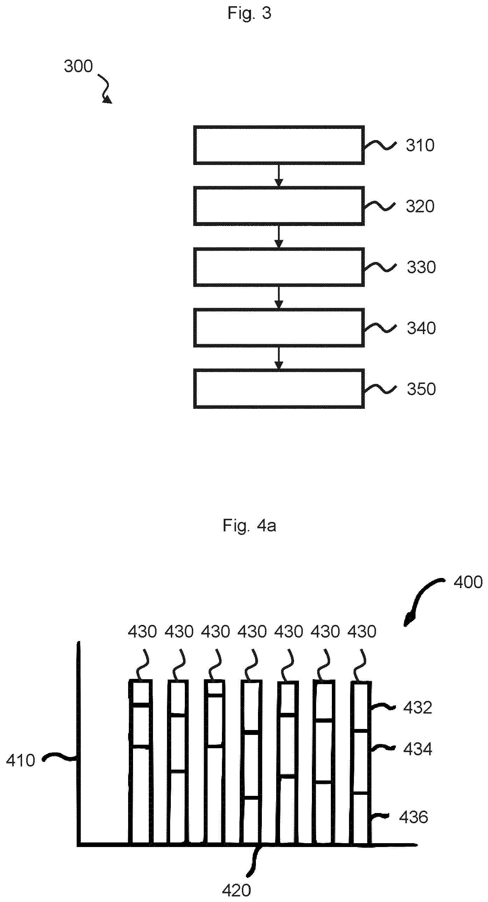

Fig. 3 zeigt ein bevorzugtes Verfahren 300. Das Verfahren 300 beginnt mit einem Schritt 310, während dem Luft in einer Luftführung entlang eines ersten Luftsensors zu einer Applikationskomponente geführt wird. Darauf folgt ein Schritt 320, während dem zumindest ein erster Luftströmungsparameter der an dem ersten Luftsensor entlang geführten Luft mittels des ersten Luftsensors erfasst wird. Darauf folgt ein Schritt 330, während dem der erste Luftsensor mittels einer Steuereinrichtung derart gesteuert wird, dass ein zeitlicher Verlauf des ersten Luftströmungsparameters als ein erstes Messergebnis gemessen wird. Darauf folgt ein Schritt 340, während dem eine Depositionsbestimmung anhand des ersten Messergebnisses, zumindest eines Depositionsparameters und eines Depositionsmodells durchgeführt wird. Das in Fig. 3 dargestellte Verfahren 300 endet mit einem Schritt 350, während dem ein Depositionsergebnis als Ergebnis der während des Schrittes 340 durchgeführten Depositionsbestimmung ausgegeben wird.

Der zumindest eine Depositionsparameter kann patientenspezifisch beziehungsweise aerosolspezifisch sein. Patientenspezifische Depositionsparameter können beispielsweise das Alter, das Geschlecht, die Ethnie, das Gewicht und die medizinische Indikation des Patienten sein. Aerosolspezifische Depositionsparameter können die Größenverteilung der Aerosolpartikel, die chemische Zusammensetzung der Aerosolpartikel, deren Dichte und weitere physikalische Eigenschaften sowie Parameter zur Zusammensetzung des Aerosols aus unterschiedlichen Stoffen sein.

Durch Verwendung einer größeren Anzahl an Depositionsparametern kann die Genauigkeit der Depositionsbestimmung und damit letztlich die Genauigkeit des Depositionsergebnisses erhöht werden.

Die Depositionsparameter können eingegeben werden und/oder in der Analyseeinrichtung 105 beziehungsweise in der Auswerteeinrichtung 160 hinterlegt sein. Um einen größtmöglichen Schutz von Patientendaten zu ermöglichen, können patientenspezifische Depositionsparameter ausschließlich lokal einzugeben und hinterlegt sein. Es ist dazu ebenfalls möglich, die patientenspezifischen Depositionsparameter verschlüsselt zu übertragen. Demgegenüber können aerosolspezifische Depositionsparameter je nach dem jeweiligen Anwendungsfall von Servern des jeweiligen Herstellers abrufbar sein, um eine zügige Inbetriebnahme und eine fehlerfreie Auswertung zu ermöglichen.

Es ist ferner möglich, dass hinterlegte Depositionsparameter mit einer spezifischen Seriennummer verknüpft sind. Sodann wird eine vereinfachte Depositionsbestimmung ermöglicht, indem lediglich die Seriennummer zur Auswerteeinrichtung übertragen wird, wobei bei der Auswerteeinrichtung die Depositionsparameter anhand der Seriennummer abgerufen werden. Dadurch wird eine noch einfachere und sicherere Auswertung ermöglicht, da bei einer potentiellen unberechtigten Abfrage der Seriennummer zumindest nicht unmittelbar auf sensible patientenspezifische Daten - die hinterlegten Depositionsparameter - rückgeschlossen werden kann.

Fig. 4a zeigt ein bevorzugtes Depositionsergebnis 400. Das Depositionsergebnis 400 ist eine Darstellung eines zweidimensionalen Diagramms. Die erste Dimension ist durch eine vertikal angeordnete Anteilsachse 410 dargestellt. Die zweite Dimension ist durch eine horizontal angeordnete Aerosoldurchmesserachse 420 dargestellt. Entlang der Aerosoldurchmesserachse 420 sind sieben Balken 430 dargestellt. Die Balken 430 befinden sich an unterschiedlichen Positionen der Aerosoldurchmesserachse 420 und entsprechen damit unterschiedlichen Aerosoldurchmessern. Die Balken 430 bestehen jeweils aus einem exhalierten Anteil 432, einem Lungenanteil 434 und einem Halsanteil 436. Die drei Anteile 432, 434, 436 sind übereinander angeordnet und entsprechen damit kumulativ einem Gesamtanteil, beispielsweise einem Anteil von 100 %. Alle sieben dargestellten Balken 430 weisen denselben Gesamtanteil auf, wobei sich die einzelnen Anteile 432, 434, 436 unterscheiden können. Mittels des in Fig. 4a dargestellten Depositionsergebnisses 400 können Rückschlüsse auf die unterschiedliche Verteilung von Aerosolen in der Lunge und im Hals sowie auf den ausgeatmeten, also exhalierten Anteil 432 in Abhängigkeit von unterschiedlichen Aerosoldurchmessern geschlossen werden.

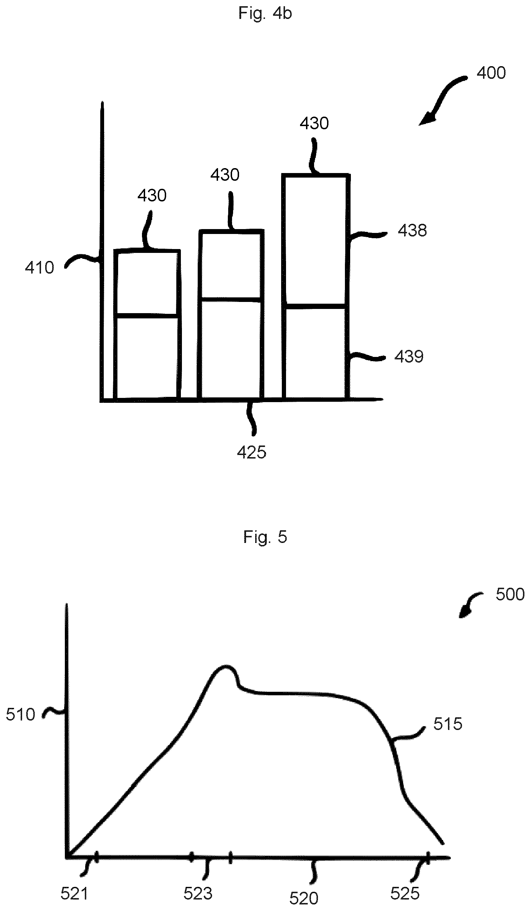

Fig. 4b zeigt ein weiteres bevorzugtes Depositionsergebnis 400. Das Depositionsergebnis 400 ist eine Darstellung eines zweidimensionalen Diagramms. Die erste Dimension ist durch eine vertikal angeordnete Anteilsachse 410 dargestellt. Die zweite Dimension ist durch eine horizontal angeordnete Inhalationsdauerachse 425 dargestellt. Entlang der Inhalationsdauerachse 425 sind drei Balken 430 dargestellt. Die Balken 430 befinden sich an unterschiedlichen Positionen der Inhalationsdauerachse 425 und entsprechen damit unterschiedlichen Inhalationsdauern. Die Balken 430 bestehen jeweils aus einem peripheren Lungenanteil 438 und einem zentralen Lungenanteil 439. Die zwei Anteile 438, 439 sind übereinander angeordnet und entsprechen damit kumulativ einem Gesamtanteil. Die drei dargestellten Balken 430 weisen jeweils einen unterschiedlichen Gesamtanteil und unterschiedliche periphere Lungenanteile 438 sowie zentrale Lungenanteile 439 auf. Mittels des in Fig. 4b dargestellten Depositionsergebnisses 400 können Rückschlüsse auf die unterschiedliche Verteilung von Aerosolen in der Lunge, insbesondere in deren peripheren und zentralen Bereich in Abhängigkeit von unterschiedlichen Inhalationsdauern geschlossen werden.

Es sind weitere Depositionsergebnisse 400 in Form von Diagrammen möglich, beispielsweise ein Balkendiagramm, bei dem jeder Inhalationszug als ein separater Balken dargestellt ist, wobei die einzelnen jeweils mit einem Inhalationszug korrespondierenden Balken entlang einer Zeitachse nebeneinander chronologisch absteigend oder aufsteigend angeordnet sind. Da eine Inhalations- beziehungsweise Therapiesitzung je nach verwendeter Apparatur und Medikamentenmenge durchaus 1.000 Inhalationszüge umfassen kann, ist es in einem solchen Fall aus Gründen der Übersichtlichkeit angebracht, als zusätzliches Depositionsergebnis 400 ein zusammenfassendes beziehungsweise kumuliertes Balkendiagramm auszugeben. Dieses kumulierte Depositionsergebnis 400 kann ebenfalls als Liniendiagramm ausgegeben werden.

Es sind außerdem weitere Depositionsergebnisse 400 möglich, beispielsweise Inhalationsbewertungen beziehungsweise Inhalationsscores, die umso besser ausfallen beziehungsweise höher liegen, je besser der Anwender sich während des Inhalierens an Zielvorgaben gehalten hat, beziehungsweise je besser der Anwender diese Zielvorgaben erreicht hat.

Ferner kann für anschließende Applikationen dem Anwender rückgemeldet werden beziehungsweise an dem Beatmungsgerät oder zusätzlichen Gerät zur kontrollierten Inhalation eingestellt werden, wie eine bessere beziehungsweise optimale Ablagerung der Medikamentenaerosole erreichbar ist. Dies kann beispielsweise dadurch geschehen, dass dem Anwender Atemmuster mit einer Folge unterschiedlich langer und intensiver Einatem- und Ausatemsequenzen vorgegeben werden. So können dem Anwender, den eine Bronchitis plagt, Atemmuster vorgegeben werden, die zu einer optimalen Ablagerung der Medikamentenaerosole in den Bronchien führen.

Das Depositionsmodell kann ferner derart erweitert werden, dass basierend auf dem Depositionsergebnis 400 die Resorption des Wirkstoffs beziehungsweise der Wirkstoffe im Körper berechnet wird.

Die Steuereinrichtung 130 kann ferner dazu eingerichtet sein, den ersten Luftsensor 120 derart zu steuern, dass eine Vielzahl aufeinanderfolgender Teilmessergebnisse messbar sind. Derart kann der Patient während einer Inhalation kontinuierlich Rückmeldung erhalten, inwieweit das jeweils aktuelle Depositionsergebnis 400 eventuellen Zielvorgaben entspricht. Gegebenenfalls kann der Patient seinen Atemfluss anpassen, um ein besseres Depositionsergebnis 400 zu erreichen, um also das Aerosol 220 im gewünschten Umfang an die gewünschte Position zu befördern.

Fig. 5 zeigt einen zeitlichen Verlauf eines ersten Luftströmungsparameters 515 als ein bevorzugtes erstes Messergebnis 500. In der dargestellten Ausführungsform wurde der erste Luftströmungsparameter 515 beziehungsweise das erste Messergebnis 500 im Zuge der Verneblung eines Medikamentenaerosols mittels eines Verneblers gemessen. Das erste Messergebnis 500 ist eine Darstellung eines zweidimensionalen Diagramms. Die erste Dimension ist durch eine vertikal angeordnete Luftströmungsparameterachse 510 dargestellt. Die zweite Dimension ist durch eine horizontal angeordnete Zeitachse 520 dargestellt. Entlang der Zeitachse 520 sind drei Ereignisse 521, 523, 525 dargestellt. Die drei Ereignisse 521, 523, 525 befinden sich an unterschiedlichen Positionen der Zeitachse 520. Am Anfang der Zeitachse 520 befindet sich das Ereignis 521, zu dem die Verneblung begonnen wurde. Ab diesem Ereignis 521 steigt der erste Luftströmungsparameter 515. Entlang der Zeitachse 520 nach rechts folgt das Ereignis 523, das der Zeitspanne entspricht, zu der die Verneblung pausiert wurde. Zu diesem Ereignis 523 erreicht der erste Luftströmungsparameter 515 seinen Höchstwert und nimmt dann wieder ab. Danach folgt am rechten Ende der Zeitachse das Ereignis 525, zu dem die Verneblung beendet wurde. Zu diesem Ereignis 525 tendiert der erste Luftströmungsparameter 515 wieder gegen Null. Mit Hilfe des ersten Messergebnisses 500, zumindest eines Depositionsparameters und eines Depositionsmodells kann anschließend eine Depositionsbestimmung durchgeführt werden.

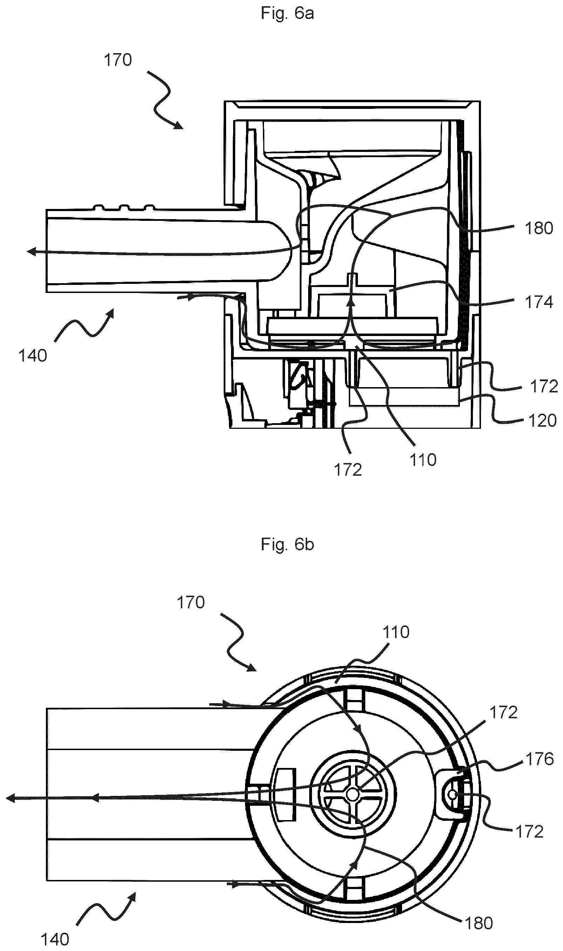

Fig. 6a zeigt eine Kopfeinheit 170 mit Applikationskomponente 140 in einer seitlichen Schnittansicht mit eingezeichneter Luftströmung 180. Die Luftströmung 180 tritt seitlich an der Applikationskomponente 140 mittels eines Bypassprinzips in den unteren Bereich der Kopfeinheit 170 ein. Die Luftführung 110 ist in der dargestellten Ausführungsform unterhalb der Kopfeinheit 170 angeordnet. Die Luftströmung 180 wird in der Luftführung 110 entlang des ersten Luftsensors 120 geführt, wobei dieser über zwei Sensorzuführungen 172 mit der Luftführung 110 verbunden ist. Nachdem die Luftströmung 180 entlang des ersten Luftsensors 120 geströmt ist, tritt sie nach oben hin über ein Auslassventil 174 zentral in die Kopfeinheit 170 ein. Daraufhin tritt die Luftströmung 180 dann über die Applikationskomponente 140 der linksseitig nach außen.

Fig. 6b zeigt die in Fig. 6a gezeigte Kopfeinheit 170 in einer Schnittansicht von unten. Die Luftströmung 180 tritt über die Außenseiten der Kopfeinheit 170 in diese ein, um dann mittig gebündelt über die Applikationskomponente 140 nach außen geleitet zu werden. Die auf der rechten Seite angeordnete Sensorzuführung 172 ist durch eine Blende 176 abgeschirmt. Die mittig angeordnete Sensorzuführung 172 ist innerhalb der Luftströmung 180 angeordnet, während die rechts angeordnete Sensorzuführung 172 mittels der Blende 176 von der Luftströmung 180 abgeschirmt ist. Derart wird eine Messung des Differenzdruckes ermöglicht, der sich einmal aus einem durch die Luftströmung 180 beeinflussten Druck - mittig angeordnete Sensorzuführung 172 - und einmal aus einem durch die Luftströmung 180 nicht direkt beeinflussten Druck - rechts angeordnete Sensorzuführung 172 - ergibt. Anhand des auf diese Weise ermittelten Differenzdruckes kann der Inhalationsstrom bestimmt werden.

Neben dem in den Fig. 6a und 6b dargestellten Messprinzip sind weitere denkbar, unter anderem die thermische Luftmassenmessung mittels beispielsweise eines gegebenenfalls als Bypass ausgebildeten, direkt im Hauptfluss angeordneten Hitzedrahtanemometers; die Differenzdruckmessung über zwei Absolutdrucksensoren zwischen zwei Messstellen im Hauptfluss, insbesondere mit Strömungswiderstand zwischen beiden Messstellen; die Differenzdruckmessung über zwei Absolutdrucksensoren, wobei ein Absolutdrucksensor in der Luftführung 110 angeordnet ist und ein zweiter gegen die Atmosphäre misst; und die Differenzdruckmessung mittels eines einzelnen im Strömungskanal angeordneten Absolutdrucksensors, wobei die Atmospährendruckänderung vernachlässigt wird. Ferner mögliche Strömungsmessprinzipien sind der mechanische Volumenzähler, die Schwebekörper- und Differenzdruckmessung, und Ultraschall-, Coriolis-, magnetischinduktive sowie thermische Durchflussmesser.

### Bezugszeichenliste

- 100: Analysesystem
- 105: Analyseeinrichtung
- 110: Luftführung
- 120: erster Luftsensor
- 122: zweiter Luftsensor
- 130: Steuereinrichtung
- 140: Applikationskomponente
- 150: Inhalator
- 160: Auswerteeinrichtung
- 170: Kopfeinheit
- 172: Sensorzuführung
- 174: Auslassventil
- 176: Blende
- 180: Luftströmung
- 210: Luft
- 220: Aerosol
- 230: Atmungsapparat
- 300: Verfahren
- 310: Schritt
- 320: Schritt
- 330: Schritt
- 340: Schritt
- 350: Schritt
- 400: Depositionsergebnis
- 410: Anteilsachse
- 420: Aerosoldurchmesserachse
- 425: Inhalationsdauerachse
- 430: Balken
- 432: Exhalierter Anteil
- 434: Lungenanteil
- 436: Halsanteil
- 438: Peripherer Lungenanteil
- 439: Zentraler Lungenanteil
- 500: Erstes Messergebnis
- 510: Luftströmungsparameterachse
- 515: Erster Luftströmungsparameter
- 520: Zeitachse
- 521: Ereignis
- 523: Ereignis
- 525: Ereignis

## Patentansprüche

1. Analysesystem (100) zur Depositionsbestimmung eines Aerosols (220) für einen Atmungsapparat (230) mit einer Analyseeinrichtung (105) aufweisend eine Luftführung (110), einen ersten Luftsensor (120), eine Steuereinrichtung (130) und eine Applikationskomponente (140), wobei
die Luftführung (110) dazu eingerichtet ist, Luft (210) entlang des ersten Luftsensors (120) zu der Applikationskomponente (140) und umgekehrt zu führen,
der erste Luftsensor (120) dazu eingerichtet ist, zumindest einen ersten Luftströmungsparameter der an dem ersten Luftsensor (120) entlang geführten Luft (210) zu erfassen, und
die Steuereinrichtung (130) dazu eingerichtet ist, den ersten Luftsensor (120) derart zu steuern, dass ein zeitlicher Verlauf des ersten Luftströmungsparameters als ein erstes Messergebnis (500) messbar ist, und das erste Messergebnis (500) zu einer Auswerteeinrichtung (160) übertragbar ist, wobei das Analysesystem (100) die Auswerteeinrichtung (160) aufweist, und wobei die Auswerteeinrichtung (160) dazu eingerichtet ist, anhand des ersten Messergebnisses (500), zumindest eines Depositionsparameters, und eines Depositionsmodells eine Depositionsbestimmung durchzuführen und als Ergebnis der Depositionsbestimmung ein Depositionsergebnis (400) auszugeben, **dadurch gekennzeichnet, dass** das Depositionsergebnis (400) anzeigt, in welchem Umfang und an welcher Position in dem Atmungsapparat (230) Medikamentenaerosole deponiert wurden.

2. Analysesystem (100) nach Anspruch 1, wobei die Steuereinrichtung (130) ferner dazu eingerichtet ist, das erste Messergebnis (500) zu speichern, und das erste Messergebnis (500) von der Steuereinrichtung (130) zu der Auswerteeinrichtung (160) übertragbar ist.

3. Analysesystem (100) nach einem der vorhergehenden Ansprüche, wobei die Steuereinrichtung (130) ferner dazu eingerichtet ist, den ersten Luftsensor (120) derart zu steuern, dass eine Vielzahl aufeinanderfolgender Teilmessergebnisse messbar sind.

4. Analysesystem (100) nach einem der vorhergehenden Ansprüche, wobei das Analysesystem (100) ferner einen zweiten Luftsensor (122) aufweist, der in der Luftführung (110) angeordnet ist, und wobei die Steuereinrichtung (130) ferner dazu eingerichtet ist, den zweiten Luftsensor (122) derart zu steuern, dass ein zeitlicher Verlauf des zweiten Luftströmungsparameters als ein zweites Messergebnis messbar ist.

5. Analysesystem (100) nach einem der Ansprüche 1 bis 3, wobei der erste Luftsensor (120) ferner dazu eingerichtet ist, einen zweiten Luftströmungsparameter der an dem ersten Luftsensor (120) entlang geführten Luft (210) zu erfassen, und wobei die Steuereinrichtung (130) ferner dazu eingerichtet ist, den ersten Luftsensor (120) derart zu steuern, dass ein zeitlicher Verlauf des zweiten Luftströmungsparameters als ein zweites Messergebnis messbar ist.

6. Analysesystem (100) nach einem der vorhergehenden Ansprüche ferner aufweisend einen Inhalator (150), wobei der Inhalator (150) insbesondere ein Vernebler, besonders bevorzugt ein Meshvernebler ist.

7. Verfahren (300) zur Depositionsbestimmung eines Aerosols (220) für einen Atmungsapparat (230) aufweisend die Schritte des:
i. Führens von Luft (210) in einer Luftführung (110) entlang eines ersten Luftsensors (120) zu einer Applikationskomponente (140);
ii. Erfassens zumindest eines ersten Luftströmungsparameters der an dem ersten Luftsensor (120) entlang geführten Luft (210) mittels des ersten Luftsensors (120);
iii. Steuerns des ersten Luftsensors (120) mittels einer Steuereinrichtung (130) derart, dass ein zeitlicher Verlauf des ersten Luftströmungsparameters als ein erstes Messergebnis (500) gemessen wird;
iv. Durchführens einer Depositionsbestimmung anhand des ersten Messergebnisses (500), zumindest eines Depositionsparameters und eines Depositionsmodells; und
v. Ausgebens eines Depositionsergebnisses (400) als Ergebnis der während Schritt iv. durchgeführten Depositionsbestimmung, **dadurch gekennzeichnet, dass** das Depositionsergebnis (400) anzeigt, in welchem Umfang und an welcher Position in dem Atmungsapparat (230) Medikamentenaerosole deponiert wurden.

8. Verfahren (300) nach Anspruch 7, wobei zwischen den Schritten iii. und iv. das erste Messergebnis (500) mittels einer Auswerteeinrichtung (160) empfangen wird und Schritt iv. mittels der Auswerteeinrichtung (160) durchgeführt wird.

9. Verfahren (300) nach Anspruch 8, wobei während Schritt iii. das erste Messergebnis (500) nach der Messung in der Steuereinrichtung (130) gespeichert wird und anschließend von der Steuereinrichtung (130) zu der Auswerteeinrichtung (160) übertragen wird.

10. Verfahren (300) nach einem der Ansprüche 7 bis 9, wobei während Schritt iii. der erste Luftsensor (120) mittels der Steuereinrichtung (130) derart gesteuert wird, dass eine Vielzahl aufeinanderfolgender Teilmessergebnisse gemessen werden.

11. Verfahren (300) nach Anspruch 10, wobei während Schritt iv. zu einer Vielzahl aufeinander folgender Zeitpunkte jeweils mit den bis zu dem aktuellen Zeitpunkt gemessenen Teilmessergebnissen eine Depositionsbestimmung durchgeführt wird und während Schritt v. zu der Vielzahl aufeinander folgender Zeitpunkte jeweils mit den bis zu dem aktuellen Zeitpunkt während Schritt iii. gemessenen Teilmessergebnissen ein aktuelles Depositionsergebnis (400) ausgegeben wird.

12. Verfahren (300) nach einem der Ansprüche 7 bis 11, wobei während Schritt i. ferner Luft (210) entlang eines zweiten Luftsensors (122) zu der Applikationskomponente (140) geführt wird, während Schritt ii. ferner ein zweiter Luftströmungsparameter der an dem zweiten Luftsensor (122) entlang geführten Luft (210) mittels des zweiten Luftsensors (122) erfasst wird, während Schritt iii. ferner der zweite Luftsensor (122) mittels der Steuereinrichtung (130) derart gesteuert wird, dass ein zeitlicher Verlauf des zweiten Luftströmungsparameters als ein zweites Messergebnis gemessen wird, und während Schritt iv. die Depositionsbestimmung zusätzlich anhand des zweiten Messergebnisses durchgeführt wird.

13. Verfahren (300) nach einem der Ansprüche 7 bis 11, wobei während Schritt ii. ferner ein zweiter Luftströmungsparameter der an dem ersten Luftsensor (120) entlang geführten Luft (210) mittels des ersten Luftsensors (120) erfasst wird, während Schritt iii. ferner der erste Luftsensor (120) mittels der Steuereinrichtung (130) derart gesteuert wird, dass ein zeitlicher Verlauf des zweiten Luftströmungsparameters als ein zweites Messergebnis gemessen wird, und während Schritt iv. die Depositionsbestimmung zusätzlich anhand des zweiten Messergebnisses durchgeführt wird.

14. Verwendung eines Analysesystems (100) nach einem der Ansprüche 1 bis 6 zur Durchführung eines Verfahrens (300) nach einem der Ansprüche 7 bis 13, insbesondere für eine menschliche Lunge.

## Claims

1. An analysis system (100) for determining the deposition of an aerosol (220) for a respiratory device (230), comprising an analysis unit (105) having an air duct (110), a first air sensor (120), a control unit (130), and an application component (140), wherein
the air duct (110) is configured to guide air (210) along the first air sensor (120) to the application component (140) and vice versa,
the first air sensor (120) is configured to detect at least a first air flow parameter of the air (210) guided along the first air sensor (120), and
the control unit (130) is configured to control the first air sensor (120) such that a temporal profile of the first air flow parameter can be measured as a first measurement result (500), and the first measurement result (500) can be transmitted to an evaluation device (160), wherein the analysis system (100) comprises the evaluation device (160), and wherein
the evaluation device (160) is configured to perform a deposition determination based on the first measurement result (500), at least one deposition parameter, and a deposition model and to output a deposition result (400) as the result of the deposition determination,
**characterized in that**
the deposition result (400) indicates the extent to which and the location within the respiratory device (230) where drug aerosols have been deposited.

2. The analysis system (100) according to claim 1, wherein the control unit (130) is further configured to store the first measurement result (500), and the first measurement result (500) can be transmitted from the control unit (130) to the evaluation unit (160).

3. The analysis system (100) according to any one of the preceding claims, wherein the control unit (130) is further configured to control the first air sensor (120) such that a plurality of successive partial measurement results can be measured.

4. The analysis system (100) according to any one of the preceding claims, wherein the analysis system (100) further comprises a second air sensor (122) arranged in the air duct (110), and wherein the control device (130) is further configured to control the second air sensor (122) in such a way that a temporal profile of the second air flow parameter can be measured as a second measurement result.

5. Analysis system (100) according to any one of claims 1 to 3, wherein the first air sensor (120) is further configured to detect a second air flow parameter of the air (210) flowing past the first air sensor (120), and wherein the control device (130) is further configured to control the first air sensor (120) such that a temporal profile of the second air flow parameter can be measured as a second measurement result.

6. An analysis system (100) according to any one of the preceding claims, further comprising an inhaler (150), wherein the inhaler (150) is, in particular a nebulizer, and more preferably a mesh nebulizer.

7. A method (300) for determining the deposition of an aerosol (220) for a respiratory device (230), comprising the steps of:
i. guiding air (210) in an air duct (110) past a first air sensor (120) to an application component (140);
ii. detecting at least a first airflow parameter of the air (210) guided along the first air sensor (120) by means of the first air sensor (120);
iii. controlling the first air sensor (120) by means of a control device (130) such that a temporal profile of the first air flow parameter is measured as a first measurement result (500);
iv. performing a deposition determination based on the first measurement result (500), at least one deposition parameter, and a deposition model; and
v. outputting a deposition result (400) as the result of the deposition determination performed during step iv.,
**characterized in that**
the deposition result (400) indicates to which extent and at which location within the respiratory device (230) drug aerosols have been deposited.

8. The method (300) according to claim 7, wherein, between steps iii. and iv., the first measurement result (500) is received by an evaluation device (160), and step iv. is performed by the evaluation device (160).

9. The method (300) according to claim 8, wherein, during step iii., the first measurement result (500) is stored in the control unit (130) after the measurement and is subsequently transmitted from the control unit (130) to the evaluation unit (160).

10. The method (300) according to any one of claims 7 to 9, wherein, during step iii., the first air sensor (120) is controlled by the control device (130) such that a plurality of successive partial measurement results are measured.

11. The method (300) according to claim 10, wherein, during step iv, a deposition determination is performed at a plurality of successive time points, in each case using the partial measurement results measured up to the current time point, and during step v, a current deposition result (400) is output at the plurality of successive time points, in each case using the partial measurement results measured up to the current time point during step iii, a current deposition result (400) is output.

12. Method (300) according to any one of claims 7 to 11, wherein, during step i, air (210) is further directed along a second air sensor (122) to the application component (140), and during step ii, a second air flow parameter of the air (210) directed along the second air sensor (122) is further detected by means of the second air sensor (122), during step iii, further, the second air sensor (122) is controlled by the control device (130) such that a temporal profile of the second air flow parameter is measured as a second measurement result, and during step iv, the deposition determination is additionally performed based on the second measurement result.

13. The method (300) according to any one of claims 7 to 11, wherein, during step ii, a second air flow parameter of the air (210) passing along the first air sensor (120) is further detected by the first air sensor (120), while during step iii, the first air sensor (120) is further controlled by the control device (130) such that a temporal profile of the second air flow parameter is measured as a second measurement result, and during step iv, the deposition determination is additionally performed based on the second measurement result.

14. Use of an analysis system (100) according to any one of claims 1 to 6 for carrying out a method (300) according to any one of claims 7 to 13, in particular for a human lung.

## Revendications

1. Système d'analyse (100) destiné à déterminer le dépôt d'un aérosol (220) pour un appareil respiratoire (230), comprenant un dispositif d'analyse (105) comportant un conduit d'air (110), un premier capteur d'air (120), un dispositif de commande (130) et un composant d'application (140), dans lequel
le conduit d'air (110) est conçu pour acheminer l'air (210) le long du premier capteur d'air (120) vers le composant d'application (140) et inversement,
le premier capteur d'air (120) est conçu pour détecter au moins un premier paramètre d'écoulement d'air de l'air (210) acheminé le long du premier capteur d'air (120), et
le dispositif de commande (130) est conçu pour commander le premier capteur d'air (120) de telle sorte qu'une évolution temporelle du premier paramètre d'écoulement d'air puisse être mesurée sous la forme d'un premier résultat de mesure (500), et que ce premier résultat de mesure (500) peut être transmis à un dispositif d'évaluation (160), le système d'analyse (100) comportant ledit dispositif d'évaluation (160), et ledit dispositif d'évaluation (160) étant conçu pour effectuer une détermination de dépôt, sur la base du premier résultat de mesure (500), au moins d'un paramètre de dépôt et d'un modèle de dépôt, et à fournir en tant que résultat de cette détermination un résultat de dépôt (400), **caractérisé en ce que** le résultat de dépôt (400) indique dans quelle mesure et à quel emplacement dans l'appareil respiratoire (230) des aérosols médicamenteux ont été déposés.

2. Système d'analyse (100) selon la revendication 1, dans lequel le dispositif de commande (130) est en outre conçu pour enregistrer le premier résultat de mesure (500), et le premier résultat de mesure (500) peut être transmis du dispositif de commande (130) au dispositif d'évaluation (160).

3. Système d'analyse (100) selon l'une des revendications précédentes, dans lequel le dispositif de commande (130) est en outre conçu pour commander le premier capteur d'air (120) de telle sorte qu'une pluralité de résultats de mesure partiels successifs puissent être mesurés.

4. Système d'analyse (100) selon l'une des revendications précédentes, dans lequel le système d'analyse (100) comprend en outre un deuxième capteur d'air (122) qui est disposé dans le conduit d'air (110), et dans lequel le dispositif de commande (130) est en outre conçu pour commander le deuxième capteur d'air (122) de telle sorte qu'une évolution temporelle du deuxième paramètre d'écoulement d'air puisse être mesurée sous la forme d'un deuxième résultat de mesure.

5. Système d'analyse (100) selon l'une des revendications 1 à 3, dans lequel le premier capteur d'air (120) est en outre conçu pour détecter un deuxième paramètre d'écoulement d'air de l'air (210) acheminé le long du premier capteur d'air (120), et dans lequel le dispositif de commande (130) est en outre conçu pour commander le premier capteur d'air (120) de telle sorte qu'une évolution temporelle du deuxième paramètre d'écoulement d'air puisse être mesurée sous la forme d'un deuxième résultat de mesure.

6. Système d'analyse (100) selon l'une des revendications précédentes, comprenant en outre un inhalateur (150), l'inhalateur (150) étant notamment un nébuliseur, de manière particulièrement préférée un nébuliseur en mailles.

7. Procédé (300) destiné à déterminer le dépôt d'un aérosol (220) pour un appareil respiratoire (230) comprenant les étapes consistant à :
i. acheminer de l'air (210) dans un conduit d'air (110) le long d'un premier capteur d'air (120) vers un composant d'application (140) ;
ii. détecter au moins un premier paramètre d'écoulement d'air de l'air (210) acheminé le long du premier capteur d'air (120) au moyen dudit premier capteur d'air (120) ;
iii. commander le premier capteur d'air (120) au moyen d'un dispositif de commande (130) de telle sorte qu'une évolution temporelle du premier paramètre d'écoulement d'air soit mesurée sous la forme d'un premier résultat de mesure (500) ;
iv. effectuer une détermination du dépôt sur la base du premier résultat de mesure (500), au moins d'un paramètre de dépôt et d'un modèle de dépôt ; et
v. émettre un résultat de dépôt (400) en tant que résultat de la détermination du dépôt effectuée au cours de l'étape iv., **caractérisé en ce que** le résultat de dépôt (400) indique dans quelle mesure et à quel emplacement dans l'appareil respiratoire (230) des aérosols médicamenteux ont été déposés.

8. Procédé (300) selon la revendication 7, dans lequel, entre les étapes iii. et iv., le premier résultat de mesure (500) est reçu au moyen d'un dispositif d'évaluation (160) et l'étape iv. est exécutée au moyen du dispositif d'évaluation (160).

9. Procédé (300) selon la revendication 8, dans lequel, au cours de l'étape iii., le premier résultat de mesure (500) est enregistré dans le dispositif de commande (130) après la mesure, puis transmis par le dispositif de commande (130) au dispositif d'évaluation (160).

10. Procédé (300) selon l'une des revendications 7 à 9, dans lequel, au cours de l'étape iii., le premier capteur d'air (120) est commandé par le dispositif de commande (130) de telle sorte qu'une pluralité de résultats de mesure partiels successifs soient mesurés.

11. Procédé (300) selon la revendication 10, dans lequel, au cours de l'étape iv., une détermination du dépôt est effectuée à une pluralité d'instants successifs, à chaque fois à partir des résultats de mesure partiels mesurés jusqu'à l'instant actuel, et au cours de l'étape v., un résultat de dépôt actuel (400) est émis à la pluralité d'instants successifs, à chaque fois à partir des résultats de mesure partiels mesurés jusqu'à l'instant actuel au cours de l'étape iii.

12. Procédé (300) selon l'une des revendications 7 à 11, dans lequel, au cours de l'étape i., de l'air (210) est en outre acheminé le long d'un deuxième capteur d'air (122) vers le composant d'application (140), tandis que, au cours de l'étape ii. un deuxième paramètre d'écoulement d'air de l'air (210) acheminé le long du deuxième capteur d'air (122) est en outre détecté au moyen du deuxième capteur d'air (122), tandis que, au cours de l'étape iii. le deuxième capteur d'air (122) est en outre commandé au moyen du dispositif de commande (130) de telle sorte qu'une évolution temporelle du deuxième paramètre d'écoulement d'air soit mesurée en tant que deuxième résultat de mesure, et au cours de l'étape iv., la détermination du dépôt est en outre effectuée sur la base du deuxième résultat de mesure.

13. Procédé (300) selon l'une des revendications 7 à 11, dans lequel, au cours de l'étape ii., un deuxième paramètre d'écoulement d'air de l'air (210) acheminé le long du premier capteur d'air (120) est en outre détecté au moyen du premier capteur d'air (120), au cours de l'étape iii., le premier capteur d'air (120) est en outre commandé au moyen du dispositif de commande (130) de telle sorte qu'une évolution temporelle du deuxième paramètre d'écoulement d'air est mesurée en tant que deuxième résultat de mesure, et au cours de l'étape iv., la détermination du dépôt est en outre effectuée sur la base du deuxième résultat de mesure.

14. Utilisation d'un système d'analyse (100) selon l'une des revendications 1 à 6 pour la mise en œuvre d'un procédé (300) selon l'une des revendications 7 à 13, en particulier pour un poumon humain.
